Europäisches Patentamt

European Patent Office

Office européen des brevets

(11) Publication number: 0 350 700
A2

# EUROPEAN PATENT APPLICATION

(21) Application number: 89111589.1

(51) Int. Cl.⁴: C07C 69/96 , C07C 68/00

(22) Date of filing: 26.06.89

(30) Priority: 11.07.88 US 217257

(43) Date of publication of application:
17.01.90 Bulletin 90/03

(84) Designated Contracting States:
DE FR GB IT NL

(71) Applicant: GENERAL ELECTRIC COMPANY
1 River Road
Schenectady New York 12305(US)

(72) Inventor: Chang, Tony Chin-Teh
4 Bayhill
Clifton Park New York 12065(US)

(74) Representative: Catherine, Alain
General Electric France Service de Propriété
Industrielle 18 Rue Horace Vernet
F-92136 Issy-Les-Moulineaux Cedex(FR)

(54) Preparation of organic carbonates by oxidative carbonylation using palladium-cobalt catalyst.

(57) Organic hydroxy compounds, preferably aromatic, are converted to carbonates by reaction with carbon monoxide and oxygen under pressure in the presence of elemental or chemically combined palladium, chemically combined cobalt, at least one tetraalkylammonium halide and at least one quinone, aromatic diol reduction product thereof or mixture thereof. The preferred species are palladium(II) acetate, cobalt(II) acetate, tetra-n-butylammonium bromide and 1,4-quinone or hydroquinone.

EP 0 350 700 A2

# PREPARATION OF ORGANIC CARBONATES BY OXIDATIVE CARBONYLATION USING PALLADIUM-CO-BALT CATALYST

This invention relates to the oxidative carbonylation of organic hydroxy compounds, and more particularly to the preparation of organic carbonates.

Organic carbonates are useful reagents for many purposes. In particular, aryl carbonates are either polycarbonates or may be converted thereto by transesterification with a bisphenol or the like. This method of polycarbonate preparation is of interest because it does not require the use of phosgene, a toxic and irritating gas which is ordinarily employed in stoichiometric excess for the preparation of such polycarbonates.

Various catalytic methods for the reaction of organic hydroxy compounds with carbon monoxide and oxygen to form carbonates are known. Typical methods of this type are described, for example, in U.S. Patents 4,201,721, 4,349,485 and 4,361,519. They all involve the use of a noble metal catalyst, typically elemental or chemically combined palladium, and for the most part require basic conditions. Also required as a practical matter in most instances, particularly for the formation of aromatic carbonates, are such materials as manganese or cobalt co-catalysts, drying agents such as molecular sieves, and phase transfer catalysts or solvents such as methylene chloride. Certain of these required materials, especially the bases and drying agents, interact with the catalyst and co-catalyst materials and convert them to forms not adaptable to convenient regeneration and recycle. In addition, these procedures often require vey long reaction times.

U.S. Patent 4,281,174 describes the reaction of alcohols with carbon monoxide and oxygen in the presence of a palladium catalyst, a quinone and a "redox agent" which may be a manganese or cobalt compound. The palladium catalyst is a complex containing ligands which may be amines, phosphines, arsines, stibines or halide or pseudo-halide salts. The products, however, are not carbonates but dialkyl oxalates.

The present invention provides a method for preparing organic carbonates which does not require the presence of bases, drying agents or solvents, making catalyst recycle more practical. It is adaptable to produce carbonates in respectable yield, by a relatively simple procedure, and often in a relatively short time.

Accordingly, the invention is a method for preparing an organic carbonate which comprises contacting an organic hydroxy compound with carbon monoxide and oxygen at a temperature in the range of about 70-150° C and partial pressures of carbon monoxide and oxygen of at least about 30 and about 2 atmospheres, respectively, in the presence of:

(A) elemental, catalytically active palladium or chemically combined palladium in the amount of about 1 gram-atom per 800-10,000 moles of organic hydroxy compound,

(B) chemically combined cobalt in the amount of about 0.5-5.0 gram-atoms per gram-atom of palladium,

(C) at least one tetraalkylammonium halide in the amount of about 10-100 moles per gram-atom of palladium, and

(D) at least one quinone, aromatic diol reduction product thereof, or mixture thereof in the amount of about 20-50 moles per gram-atom of palladium.

The reactants employed in the method of this invention are an organic hydroxy compound, carbon monoxide and oxygen which may be provided in the form of air. The organic hydroxy compound may be an aliphatic, alicyclic or aromatic mono- or polyhydroxy compound such as methanol, ethanol, cyclohexanol, phenol, cresol, xylenol, ethylene glycol, propylene glycol, resorcinol, hydroquinone or bisphenol A. Aromatic hydroxy compounds and especially monohydroxy compounds are preferred, with phenol being most preferred.

According to the invention, the oxidative carbonylation reaction takes place in the presence of various chemical species serving as catalysts, redox agents and the like. Component A is palladium in elemental, catalytically active form or chemically combined form. Thus, palladium black or elemental palladium deposited on carbon are suitable, as well as palladium compounds such as halides, nitrates, carboxylates and complexes involving such compounds as carbon monoxide, amines, phosphines or olefins. Preferred in most instances are palladuim(II) salts of organic acids, most often carboxylates with $C_{2-6}$ aliphatic acids. Palladium(II) acetate is most preferred.

Component B is chemically combined cobalt, which may be divalent or trivalent and is preferably divalent. Again, any cobalt compound may be used for this purpose, including simple salts such as halides and carboxylates and complexes with amines, carbon monoxide and the like. As with component A, the carboxylates and especially cobalt(II) acetate are preferred.

Component C, the tetraalkylammonium halide, is critical for the formation of organic carbonates pursuant to the invention. The chlorides and bromides and especially the bromide are preferred, and the most useful alkyl groups are primary and secondary alkyl groups containing about 1-8 carbon atoms. Most preferred is tetra-n-butylammonium bromide.

Component D is at least one quinone, aromatic diol formed by reduction of said quinone, or mixture thereof. 1,4-Quinone and hydroquinone are preferred. Other suitable compounds are 1,2-quinone and catechol, and 9,10-anthraquinone and 9,10-dihydroxyanthracene.

According to the invention, contact between the organic hydroxy compound, carbon monoxide, oxygen and other components is effected under high pressure and at a temperature in the range of about 70-150°C, preferably about 80-130°C. The partial pressures of carbon monoxide and oxygen are at least about 30 and at least about 2 atmospheres, respectively, with preferred partial pressures being in the range of about 40-60 and about 4-10 atmospheres, respectively.

The proportions of components A, B, C and D are subject to wide variation, with certain preferences for the preparation of organic carbonate in maximum yield. Thus, component A is present in the amount of about 1 gram-atom of palladium per 800-10,000 and preferably 6000-10,000 moles of organic hydroxy compound. Proportions of other constituents are in terms of gram-atom of palladium and are, respectively, about 0.5-5.0 and preferably about 1-3 gram-atoms of cobalt, about 10-100 and preferably about 50-80 moles of tetraalkylammonium halide, and about 20-50 and preferably about 25-40 moles of quinone and/or reduction product thereof.

It is within the scope of the invention to employ solvents in the reaction. Suitable solvents include aliphatic, alicyclic and aromatic hydrocarbons such as hexane, heptane, cyclohexane, toluene and decalin; halogenated hydrocarbons such as methylene chloride, chloroform and chlorobenzene; ethers such as diphenyl ether and dioxane, and esters such as ethyl acetate and methyl formate. Also contemplated is the use of drying agents such as activated alumina, calcium sulfate, calcium chloride and molecular sieves. However, under most conditions the use of solvents and drying agents offers no advantage and is therefore not preferred.

Recovery of the organic carbonate may be effected by conventional operations including, for example, distillation. The palladium may also be recovered, for example, by treatment with nitric acid and reconversion to a carboxylate or other compound particularly useful as component A.

The invention is illustrated by the following examples.

Example 1

A 300-ml. autoclave was charged with 75 grams (800 mmol.) of phenol, 23 mg. (0.1 mmol.) of palladium(II) acetate, 18 mg. (0.1 mmol.) of cobalt(II) acetate, 2 grams (6 mmol.) of tetrabutylammonium bromide and 330 mg. (3 mmol.) of hydroquinone. The autoclave was sealed, pressurized with 54.4 atmospheres of carbon monoxide and 5.4 atmospheres of oxygen, heated at 120°C for 1 hour, cooled an vented. Analysis by gas chromatography showed the presence of diphenyl carbonate in the amount of 5.4 grams, corresponding to a phenol conversion of 6.3%.

Example 2

The procedure of Example 1 was repeated, substituting quinone on a equimolar basis for the hydroquinone. The product contained 6.7 grams of diphenyl carbonate, corresponding to 8% conversion of phenol.

Example 3

The procedure of Example 1 was repeated, replacing the palladium(II) acetate with an equivalent amount of 10% palladium on carbon. The product contained 5.2 grams of diphenyl carbonate, corresponding to 6.1% conversion of phenol.

Example 4

The procedure of Example 1 was repeated, substituting bis(diphenylphosphino)methane palladium(II) chloride for the palladium(II) acetate. The product contained 5.5 grams of diphenyl carbonate, corresponding to 6.4% conversion of phenol.

Example 5

The autoclave of Example 1 was charged with 9.4 grams (100 mmol.) of phenol, 23 mg. (0.1 mmol.) of palladium(II) acetate, 53 mg. (0.3 mmol.) of cobalt(II) acetate, 480 mg. (1.5 mmol.) of tetrabutylammonium bromide, 330 mg. (3 mmol.) of hydroquinone and 30 ml. of methylene chloride. The autoclave was sealed, pressurized with 54.4 atmospheres of carbon monoxide and 13.6 atmospheres of oxygen, heated at 90°C for 5 hours, cooled and vented. Upon analysis by gas chromatography, the product was shown to contain diphenyl carbonate in an amount corresponding to 14% conversion of phenol. Upon repressurizing to the same partial pressures of carbon monoxide and oxygen and heating at 90°C for another 5 hours, the conversion was increased to 19%. The palladium in the catalyst was recovered by filtration as a black solid, capable of regeneration by treatment with nitric acid.

Example 6

The procedure of Example 5 was repeated, with the addition of 6 grams of 3A molecular sieves. The conversion to diphenyl carbonate after 5 and 9 hours was 34% and 38%, respectively.

Example 7

The procedure of Example 5 was repeated, substituting 1,2-dichloroethane for the methylene chloride and conducting the reaction for 4 hours. The conversion to diphenyl carbonate was 5%.

Example 8

The procedure of Example 5 was repeated, replacing the oxygen with air the same partial pressure (oxygen partial pressure 2.7 atmospheres) and conducting the reaction for 4 hours. The conversion to diphenyl carbonate was 11%.

Claims

1. A method for preparing an organic carbonate which comprises contacting an organic hydroxy compound with carbon monoxide and oxygen at a temperature in the range of about 70-150°C and partial pressures of carbon monoxide and oxygen of at least about 30 and about 2 atmospheres, respectively, in the presence of:

(A) elemental, catalytically active palladium or chemically combined palladium in the amount of about 1 gram-atom per 800-10,000 moles of organic hydroxy compound,

(B) chemically combined cobalt in the amount of about 0.5-5.0 gram-atoms per gram-atom of palladium,

(C) at least one tetraalkylammonium halide in the amount of about 10-100 moles per gram-atom of palladium, and

(D) at least one quinone, aromatic diol reduction product thereof, or mixture thereof in the amount of about 20-50 moles per gram-atom of palladium.

2. A method according to claim 1 wherein component A is a palladium(II) salt of an organic acid.

3. A method according to claim 2 wherein reagent A is palladium(II) acetate.

4. A method according to claim 2 wherein component B is a cobalt(II) salt of an organic acid.

5. A method according to claim 4 wherein reagent B is cobalt(II) acetate.

6. A method according to claim 4 wherein component C is a tetraalkylammonium bromide in which the alkyl groups are primary or secondary alkyl groups containing about 1-8 carbon atoms.

7. A method according to claim 6 wherein component C is tetra-n-butylammonium bromide.

8. A method according to claim 6 wherein component D is at least one of 1,4-quinone and hydroquinone.

9. A method according to claim 8 wherein oxygen is provided in the form of air.

10. A method according to claim 8 wherein the organic hydroxy compound is phenol.

11. A method according to claim 9 wherein the reaction temperature is within the range of about 80-130°C.

12. A method according to claim 11 wherein component A is present in the amount of about 1 gram-atom of palladium per 6000-10,000 moles of organic hydroxy compound, component B in the amount of about 1-3 gram-atoms of cobalt per gram-atom of palladium, component C in the amount of about 50-80 moles per gram-atom of platinum, and component D in the amount of about 25-40 moles per gram-atom of palladium.

13. A method according to claim 12 wherein the partial pressures of carbon monoxide and oxygen are in the range of about 40-60 and about 4-10 atmospheres, respectively.

14. A method according to claim 13 wherein the organic hydroxy compound is phenol.

15. A method according to claim 14 wherein reagent A is palladium(II) acetate, reagent B is cobalt(II) acetate and reagent C is tetra-n-butylammonium bromide.

16. A method according to claim 15 wherein the oxygen is provided in the form of air.